# EUROPÄISCHE PATENTSCHRIFT

(11) **EP 0 745 139 B1**
(45) Veröffentlichungstag und Bekanntmachung des Hinweises auf die Patenterteilung: **24.04.2002**
(21) Anmeldenummer: 93912550.6
(22) Anmeldetag: 19.05.1993
(51) Int. Cl.: C12Q 1/68

(54) **VERFAHREN ZUR PRÄPARIERUNG UND HYBRIDISIERUNG VON SPEZIFISCHEN PROBEN**
PROCESS FOR PREPARING AND HYBRIDIZING SPECIFIC PROBES
PROCEDE DE PREPARATION ET D'HYBRIDATION DE SONDES SPECIFIQUES

(30) Priorität: 22.05.1992 DE 4216949
(43) Veröffentlichungstag der Anmeldung: 04.12.1996
(73) Patentinhaber: CREMER, Cristoph, D-69126 Heidelberg (DE)
(72) Erfinder: CREMER, Cristoph, D-6900 Heidelberg (DE); CELEDA, Dino, D-6730 Neustadt (DE); BETTAG, Ulrich, D-6710 Frankenthal 6 (DE)
(74) Vertreter: Pietruk, Claus Peter, Dipl.-Phys.
(86) Internationale Anmeldenummer: DE9300460
(87) Internationale Veröffentlichungsnummer: WO9324652

(56) Entgegenhaltungen:
- EP-A- 0 135 159
- EP-A- 0 336 412
- EP-A- 0 450 370
- WO-A-93/05177
- THIRTIETH ANNUAL MEETING OF THE JAPAN SOCIETY OF HISTOCHEMISTRY AND CYTOCHEMISTRY, KYOTO, JAPAN, OCTOBER 23-24, 1989. ACTA HISTOCHEM CYTOCHEM 23 (3). KOJI T ET AL. 'USE OF NUCLEOTIDES AS AN ALTERNATIVE TO FORMAMIDE IN NON-RADIOACTIVE IN- SITU HYBRIDIZATION .'
- NUCLEIC ACIDS RES 20 (5). 1992. 1149-1150. CODEN: NARHAD ISSN: 0305-1048 IEZZONI J C ET AL. 'COLORIMETRIC DETECTION OF HERPES SIMPLEX VIRUS BY DNA IN- SITU SANDWICH HYBRIDIZATION A RAPID FORMAMIDE -FREE RANDOM OLIGOMER-ENHANCED METHOD.'
- J. AM. SOC Bd. 111, 1989, Seiten 3059 - 3061 POVSIC ET AL. 'Triple helix formation by oligonucleotides on DNA extended to the physiological range'
- BIOLOGICAL ABSTRACTS vol. 95, no. 4 , 1993, Philadelphia, PA, US; abstract no. 38738,

## Beschreibung

Die Erfindung bezieht sich auf ein Verfahren zur Durchführung von in situ Hybridisierungen von spezifischen Proben, insbesondere Nukleinsäuren oder Polyamidnukleinsäuren oder anderen für Hybridisierungen geeignete Proben, an nukleinsäureenthaltende chromosomale oder extrachromosomale Zielobjekte, wobei Proben und Zielobjekte in einem geeigneten wässrigen Medium in engen räumlichen Kontakt gebracht werden und das wässrige Medium denaturierende Agenzien in einer Konzentration zwischen 0% und 10% , insbesondere 0%, enthält. Als Zielobjekte werden hier chromosomale und extrachromosomale Strukturen in Eukaryonten wie z. B. menschliche Zellen, Zellen von Säugetieren und pflanzliche Zellen angesehen.

Die Analyse von Chromosomen spielt in der biologischen, medizinischen und klinischen Forschung und Diagnostik eine immer größere Rolle. Beim Studium pathologischer Prozesse wurde beispielsweise eine enge Korrelation zwischen Chromosomenmorphologie und Malignität gefunden. Verfahren zur In-situ-Hybridisierung haben deshalb bereits vielfältige Anwendungen erlangt, z. B. in der Tumorforschung, bei der Detektion mutagen-induzierter Chromosomenaberrationen und in der Erforschung des spezifischen Chromatins im Interphase-Kern.

Die in-situ-Hybridisierung, das heißt die spezifische Anlagerung von spezifischen, insbesondere Nukleinsäure-enthaltenden Proben (im folgenden einfach als Proben bezeichnet) an Nukleinsäure-enthaltende Zielobjekte, insbesondere Chromosomen, beruht darauf, daß sich komplementäre Basen (Purine, Pyrimidine oder ähnliche Moleküle) zusammenlagern. Komplementäre Nukleinsäureketten lagern sich deshalb spontan aneinander an und bilden mehrsträngige Ketten. Unter Ausnutzung dieser Eigenschaften lassen sich spezifische Sequenzen von Zielobjekten, beispielsweise Chromosomen, dadurch identifizieren und gegebenenfalls lokalisieren, daß man spezifische Proben zu dem Zielobjekt gibt, wo sie spezifisch mit der gesuchten, komplementären Sequenz hybridisieren, also sich an diese binden, sofern diese Sequenz vorhanden ist. Sind die Proben markiert, beispielsweise mit einem Fluoreszenzmarker, kann man sie nach der Hybridisierung über diesen Marker detektieren und lokalisieren.

Bisher ist es die feste Überzeugung der Fachwelt, daß eine (im folgenden ISH genannte) in situ Hybridisierung nur erfolgreich durchgeführt werden kann, wenn sowohl das Zielobjekt als auch die Proben-Desoxyribonukleinsäure (DNS) bzw. Proben-Ribonukleinsäure (RNS) weitgehend in einzelsträngiger Form vorliegen. Dies kommt in einer Vielzahl von Publikationen der letzten Jahre und Jahrzehnte klar zum Ausdruck. Sie gingen davon aus, daß in der DNS enthaltene natürliche, einzelsträngige Abschnitte, z. B. hairpins bzw. einzelsträngige Abschnitte aufgrund von Faltungen, für die ISH nicht ausreichend seien. Es wird deshalb allgemein davon ausgegangen, daß eine Zielobjekt-DNS, die normalerweise im wesentlichen in doppelsträngiger, d.h. Duplex-Form vorliegt, vor einer ISH in geeigneter Weise behandelt werden muß, um diese in eine einzelsträngige Form zu überführen. Aus diesem Grund beruhen alle für die ISH verwendeten Verfahren auf der Verwendung von Formamid oder anderen denaturierenden, die Denaturierung fördernden oder erhaltenden chemischen Agenzien siehe z.B. Koji et al. 1990, Acta Histochem Cytochem 23, S. 327-334. Auch geeignete Enzyme, wie beispielsweise Exonukleasen, werden zur Erzeugung von Einzelsträngen eingesetzt, wobei bei allen bekannten ISH-Experimenten, bei denen derartige Enzyme eingesetzt wurden, zusätzlich Formamid verwendet wurde und zwar in Konzentrationen über 30%. Hilfreich für die Denaturierung ist auch eine Erhöhung der Temperatur des das Zielobjekt enthaltenden Mediums, was als alleinige Maßnahme aber bisher als nicht ausreichend betrachtet wird.

Andere Lösungen bestehen darin, Methoden anzuwenden, bei denen die thermische Denaturierung der Zielobjekt-DNS vermieden wird. Dies wird z.B. erreicht durch entsprechende Mengenerhöhung bei dem Einsatz von denaturierenden oder die Denaturierung fördernden Agenzien. Hier sind beide als denaturierende Agenzien bezeichnet. So kann die Zielobjekt-DNS beispielsweise mit NaOH oder durch den Einsatz sehr hoher Konzentrationen von Formamid auch bei niedrigen Temperaturen in eine einzelsträngige denaturierte Form überführt werden. Empfindliche immunologische Strukturen sind gegenüber einer solchen Behandlung nicht genügend stabil. Als Alternative bieten sich Möglichkeiten eines Einsatzes enzymatischer Verfahren zur Produktion von einzelsträngiger Zielobjekt-DNS an, z. B. Exonukleasen. Jedoch wurde auch hier die Hybridisierung mit einem Formamidhaltigen Puffersystem durchgeführt, wenn auch in geringerer Konzentration (-30%), als sie bei der Erzeugung nicht-enzymatischer, einzelsträngiger Zielobjekt-DNS bei niedrigen Temperaturen für erforderlich gehalten wurde.

Ein seit vielen Jahren aufgrund von Untersuchungen an synthetischen Nukleinsäuren postulierter Mechanismus besteht in der Verbindung von einzelsträngigen Nukleinsäuren mit doppelsträngigen Duplex-Nukleinsäuren zu dreisträngigen Triple-Formationen. Bei der Triple-Formation sind drei Nukleinsäurestränge in unmittelbarer Nachbarschaft zueinander angeordnet. Triplex-Strukturen bilden sich bevorzugt dann, wenn in der Zielobjekt-DNS Homo-Pyrimidinabschnitte bestimmter Länge vorliegen. Alle dreisträngigen Formen von Nukleinsäuren bzw. Basensträngen sind hier unter dem Begriff Triple-Formation zusammengefaßt.

Ferner ist es bekannt, daß auch gemischte Sequenzen aus Purinbasen und Pyrimidinbasen bei der Triplebildung zulässig sind. Bei nicht-enzymatischen Hybridisierungsverfahren bei niedrigen Temperaturen (T < 5°C) wurde festgestellt, daß Triplebildungen, d. h. doppelsträngige Proben und einzelsträngige Proben, auch in Abwesenheit denaturierender Agenzien erfolgen können. Dies hält man bisher allerdings ausschließlich bei Hybridisierungen in vitro mit isolierter DNS für möglich.

Es wird zwar angenommen, daß auch in der nativen DNS einzelsträngige Abschnitte existieren könnten bzw. die Anlagerung einzelsträngiger DNS/doppelsträngiger DNS, z.B. in Form einer Triplex, erfolgen könnte. Ferner ist es bekannt, daß einzelsträngige Proben, in denen das Zucker-Phosphat-Rückgrat durch eine Polyamidkette ersetzt worden war (sogenannte PNS), sich in einer sequenzspezifischen Weise mit doppelsträngiger DNS zu einer neuen Form dreisträngiger Formationen- der DNS-PNS- verbinden können.
Noch niemals wurden jedoch hieraus Konsequenzen für die Möglichkeit eines Verfahrens zur nicht-enzymatischen ISH ohne Verwendung denaturierender Agenzien gezogen.

Die Meinung, daß denaturierende Agenzien für eine erfolgreiche ISH absolut notwendig seien, ist so stark, daß selbst Autoren, die eine ISH bei niedrigen Temperaturen nach enzymatischer Behandlung durchführten, anschließend noch zusätzliche denaturierende Agenzien, im speziellen Fall Formamid, verwendeten. Die Vorstellung, daß die doppelsträngige DNS vor einer Hybridisierung durch entsprechende Behandlung in einzelsträngige Zielobjekt-DNS überführt werden müsse, ist offensichtlich so stark verankert, daß keine Kontrollexperimente durchgeführt wurden, in denen auf eine Behandlung mit Formamid verzichtet wurde.

Durch Verwendung von Formamid wird drastisch die Schmelztemperatur von doppelsträngiger DNS (ds-DNS) gesenkt. Weiterhin kann man auf der Basis energetischer Betrachtungen erwarten, daß in Gegenwart von Formamid u. U. die Bildung von Triplex-DNS sogar noch stärker gestört sein könnte als die Bildung von ds-DNS.

Chromosomale Proteine sind von erheblicher Bedeutung für die Tertiärstruktur der Chromosomen sowie der chromosomalen DNS. Veränderungen der Tertiärstruktur chromosomaler Proteine könnten daher die Konformation der chromosomalen DNS erheblich beeinflussen, z. B. die Ausbildung von Triple-Formationen. Da die Tertiärstruktur von Proteinen durch denaturierende Agenzien erheblich gestört werden kann, könnten Formamid und andere denaturierende Agenzien auch in dieser Hinsicht einen die ISH letztlich behindernden Einfluß haben. Ähnliche Überlegungen wie für Formamid gelten auch für andere denaturierende Agenzien.

Die bekannten ISH-Verfahren haben erhebliche Nachteile. Denaturierende Substanzen wie z.B. Formamid üben ihre denaturierende Wirkung nicht selektiv auf Nukleinsäuren aus, sondern auch auf andere Bestandteile der Zelle, wie beispielsweise Proteine. Immuntechnische Färbemethoden (IT) lassen sich deshalb in Gegenwart solcher denaturierender Agenzien wegen deren Wirkung auch auf immunologisch relevante Strukturen nur sehr begrenzt durchführen. Deshalb ist es auch meist nicht, oder nur mit größerem Aufwand möglich, ISH-Verfahren mit immunologischen Färbeverfahren zu kombinieren, obwohl dies insbesondere in der klinischen Diagnostik im Interesse einer möglichst schnellen und sicheren Diagnose sehr wünschenswert wäre. Alle bekannten Verfahrensansätze, um zu einer kombinierten Charakterisierung von Zellen durch IT und ISH zu kommen, sind jedoch auf relativ seltene, natürlich vorkommende, gegenüber thermischer Denaturierung und/oder Behandlung mit denaturierenden Agenzien ausreichend resistente, immunologisch relevante Strukturen beschränkt oder auf die präparative Erzeugung resistenter, immunologischer Strukturen. Eine praktizierte Methode zur Kombination von ISH und IT ist die, daß zunächst eine IT-Reaktion mit Antikörpern durchgeführt wird, die biotinyliert oder mit anderen hitzebeständigen und resistenten Gruppen versehen worden sind, und anschließend die ISH-Prozeduren durchgeführt werden. Diese Methode ist aber relativ langwierig und teuer.

Ein allgemeiner Weg zur Realisierung einer gleichzeitigen Charakterisierung von Zellen durch immunologische Techniken (IT) und ISH-Methoden von chromosomalen Zielobjekten wurde bei den derzeit verwendeten ISH-Verfahren bisher nicht gesehen, da die hierbei benutzten Präparationsschritte die immunologische Färbung wesentlich beeinträchtigen.

Aufgabe der Erfindung ist es, ein ISH-Verfahren zur Verfügung zu stellen, das einfacher, schneller und ökonomischer durchführbar ist als konventionelle Verfahren, und bei dem die Zielobjekte wie beispielsweise Chromosomen, Zellen oder Gewebe schonender behandelt werden, wodurch die Kombination des ISH-Verfahrens mit immunologischen Techniken möglich wird.

Gelöst wird die Aufgabe erfindungsgemäß dadurch, daß zur Durchführung des ISH-Verfahrens die Proben und Zielobjekte in einem geeigneten wässrigen Medium in engen räumlichen Kontakt gebracht werden,
- daß die Zielobjekte ohne denaturierende Vorbehandlung eingesetzt werden,
- daß die Proben als Einzel- und/oder Doppelstränge eingesetzt werden, wobei die Einzelstränge originär erzeugt oder sekundär durch Hitzedenaturierung aus ursprünglichen Doppelsträngen hergestellt sind,
- daß das wässrige Medium bzw. der Puffer frei von denaturierenden die Denaturierung fördernden oder erhaltenden chemischen Agenzien und Enzymen in Mengen, die für eine Behandlung zur Erzeugung von einzelsträngigen DNS-Abschnitten geeignet sind, ist,
- daß Zielobjekte, Proben und wässriges Medium zur Hybridisierungsmixtur miteinander vermischt werden, und
- daß die Hybridisierungsphase bei einer Temperatur zwischen +2°C und +100°C durchgeführt wird, wobei die Hybridisierungsmixtur frei ist von denaturierenden die Denaturierung fördernden oder erhaltenden chemischen Agenzien und Enzymen in Mengen, die für eine Behandlung zur Erzeugung von einzelsträngigen DNS-Abschnitten geeignet sind

Auf diese Weise ist eine erhebliche Vereinfachung und Reduzierung der Verfahrensschritte möglich. Wesentlich ist hierbei, daß denaturierende Agenzien vollständig weggelassen, oder nur in relativ geringen Mengen von maximal 10% zugesetzt werden, die auch empfindliche Strukturen nicht negativ beeinflussen. Hierdurch wird eine schonendere Behandlung der Zielobjekte bzw. der Targets erreicht, und zwar insbesondere deshalb, weil aufwendige Verfahrens- und Waschschritte, gekoppelt an den Gebrauch höherer Konzentrationen an denaturierenden Agenzien, die bisher zwingend notwendig erschienen, eingespart werden können. Aus der Reduktion der Verfahrensschritte sowie aus der Zeitersparnis resultiert darüberhinaus eine gute Morphologie des chromosomalen Materials in Verbindung mit einer guten Visualisierung der markierten Proben. Ferner kann mit dem erfindungsgemäßen Verfahren eine bessere und raschere Detektierung mittels quantitativer Bildanalyseverfahren, erfolgen. Insbesondere kann eine geringere statistische Schwankung der intrazellulären Hybridisierungssignalverhältnisse erreicht werden, wobei das erfindungsgemäße Verfahren in sehr kurzer Zeit, das heißt, in weniger als 30 min, in vielen Fällen sogar in weniger als 5 min, realisiert werden kann. Durch seine Einfacheit und Schnelligkeit ist das Verfahren auch geeignet, größere Mengen an Material in sehr kurzer Zeit zu untersuchen bzw. auszuwerten. Es lassen sich insbesondere statistische Untersuchungsmethoden optimal einsetzen, z.B. in der Erfassung strahleninduzierter bzw. chemisch induzierter Chromosomenveränderungen im Niedrigdosisbereich. Es kann so auf erheblich einfachere und schnellere Weise der Einfluß geringer Strahlungsdosen an den Chromosomen nachgewiesen werden, so daß sich Aberrationsraten bzw. Chromosomenveränderungen bestimmen lassen.

Von besonderem Vorteil ist es, daß man bei der ISH vollständig ohne denaturierende Agenzien, z. B. Formamid, und auch ohne enzymatische Behandlung auskommen kann und daß die chromosomalen Zielobjekte Temperaturen über +45°C nicht ausgesetzt werden müssen (vgl. Anspruch 2). Denaturierende Agenzien bzw. enzymatische Behandlungen zur Erzeugung einzelsträngiger DNS-Abschnitte sind bei der Durchführung des erfindungsgemäßen ISH-Verfahrens nicht mehr erforderlich; dies gilt auch für physikalische, denaturierende Agenzien, z. B. UV-Licht und/oder ionisierende Strahlung.

Das erfindungsgemäße Verfahren führt zu für spezifische ISH charakteristischen Markierungen, obwohl während der gesamten ISH-Prozedur das Zielobjekt nicht mit denaturierenden Agenzien behandelt wurde. Der Zusatz geringer Mengen denaturierender Agenzien hat in vielen Fällen keinen negativen Einfluß, sofern deren Konzentration unter 10% bleibt.

Dies zeigt, daß die seit Jahrzehnten herrschende Überzeugung der Fachwelt, daß nämlich eine erfolgreiche ISH nur unter Verwendung denaturierender Agenzien in höheren Konzentrationen, das bedeutet in der Praxis Konzentrationen über 30%, entsprechender physikalischer Maßnahmen oder entsprechender Enzyme, wie beispielsweise Exonukleasen, funktionieren karm, überaschenderweise nicht richtig ist. Offensichtlich reichen, entgegen der herrschenden Lehrmeinung, die an sich bekannten Triplebildungen aus einzel- und doppelsträngigen Nukleinsäureketten, sowie die natürlicherweise vorkommenden einzelsträngigen Chromosomenabschnitte aus, um geeignete Proben zuverlässig an spezifische Stellen von Zielobjekten zu hybridisieren. Zielobjekte können bei der neuartigen ISH also einzel-, doppel- oder mehrsträngig und nach der Hybridisierung doppel- oder mehrsträngig sein.

Mit Hilfe von Antikörpern gegen Triple-DNS wurde gezeigt, daß derartige Triple-Formationen in Chromosomen verschiedener Eukaryontenspezies, insbesondere auch in Chromosomen menschlicher Zellen, weit verbreitet sind.

Proteine können für einzelsträngige, doppelsträngige oder auch für Triple-DNS sequenzspezifisch sein und können auch Bindungsstellen beispielsweise für doppelsträngige-DNS haben. Hieraus ergeben sich weitere grundsätzliche Möglichkeiten einer In-situ-Hybridisierung. Zum Beispiel bindet ein Protein sequenzspezifisch an einen chromosomalen DNS-Abschnitt und in sequenzspezifischer Weise die Proben-Nukleinsäure. Auch Dimerisierungsmodelle, zwei sequenzspezifische Proteine, die sich zusammenlagern, sind realisierbar. Als Proben können beispielsweise auch spezifische Antikörper verwendet werden (vgl. Anspruch 10).

Eine wichtige Rolle für eine erfolgreiche ISH spielt die Temperatur, bei der das Verfahren durchgeführt wird. Die optimale Temperatur der Hybridisierungsmixtur hängt davon ab, welches Ziel mit der ISH verbunden ist. Prinzipiell ist das neuartige ISH-Verfahren bei beliebigen Temperaturen zwischen +2°C und +100°C durchführbar. Insbesondere bei Kombination der ISH mit IT ist es vorteilhaft, daß die Hybridisierungsmixtur, insbesondere das Zielobjekt, mit relativ niedrigen Temperaturen zwischen +2°C und +45°C, insbesondere zwischen +2°C und +37°C, ganz bevorzugt zwischen +18°C und +28°C behandelt werden kann (vgl. Anspruch 2). Mit anderen Worten bedeutet dies, das die ISH auch bei Raumtemperatur durchgeführt werden kann, daß also auch auf eine thermische Denaturierung des Zielobjektes verzichtet werden kann, wodurch auch sehr empfindliche immunologisch relevante Strukturen unbeschadet bleiben. Die ISH wird bei einer solchen Kombination von ISH und IT durch Zugabe der Probe, die vorher gegebenenfalls thermisch behandelt wird, gestartet. Dieses extrem schonende Verfahren wird auch bevorzugt dann eingesetzt, wenn durch die Hybridisierung eine Blockade bestimmter Gensequenzen durch die Probe bezweckt wird.

Auch eine Stringenzregelung bzw. Verhinderung der Bindung von Proben an chromosomale Zielobjekte geringerer Komplementarität kann durch eine geeignete Einstellung der Hybridisierungstemperatur erfolgen.

Unter dem Begriff "Hybridisierungsmixtur" wird hier das wässrige Medium verstanden, das mindestens die Proben und die Zielobjekte enthält.

Eine gleichzeitige Evaluierung von Präparaten nach In-situ-Hybridisierungs-Parametern und immunologischen Parametern, z. B. Oberflächenantigenen, ist für die klinische Diagnostik von großer Bedeutung. In weiterer Ausgestaltung der Erfindung ist es vorteilhaft, daß die Analyse der Präparate gleichzeitig nach Oberflächenantigenen und Chromosomenveränderungen mit beliebiger Länge der markierten Zielobjekt-Sequenz bei mikroskopischer Beobachtung und/oder digitaler Mikroskopie und/oder flußzytometrischen Techniken oder dergleichen erfolgt, dergestalt, daß die Zelle weitgehend unbeschädigt bleibt und gleichzeitig die Zielobjekte gut kenntlich gemacht werden können (vgl Ansprüche 13 und 14). Hierdurch lassen sich die einzelnen Zielobjekte in sehr kurzer Zeit klassifizieren.

Für eine gleichzeitige immunologische Färbung und Niedertemperatur-ISH (Hybridisierungstemperatur < +45°C) wird ein Verfahren angewandt, das eine ausreichende Permeabilisierung der Zellmembran zuläßt, ohne sie zu zerstören und ohne die Antigen-Expression der Zelloberfläche entscheidend zu verändern. Hierzu können beispielsweise Detergentien im Medium enthalten sein (vgl. Anspruch 8).

Bei der erfindungsgemäßen Niedertemperatur-ISH wurden die Chromosomen mit Fixierverfahren (Methanol/Eisessig; Glutaraldehyd, Formaldehyd etc.), behandelt. Letzieres stellt jedoch kein Denaturierungsverfahren dar und ist für den Hybridisierungsvorgang als solchen nicht erforderlich. Auf eine solche Fixierung kann auch verzichtet werden.

Es ist erfindungswesentlich, daß auch bei niedrigen Temperaturen unter +28°C die außerordentlich stabilen ds-DNS-PNS-Komplexe zu einer effektiven ISH führen, auch wenn die Zahl der Basen der PNS-Sequenzen, die zu branchbaren ds-DNS-PNS-Komplexen führen, relativ klein bleibt.

Je nach den Gegebenheiten ist es möglich, das ISH-Verfahren mit einzel- oder doppelsträngigen Proben durchzuführen. Bevorzugt werden jedoch einzelsträngige Proben (vgl. Anspruch 5). Liegen die Proben zunächst in doppelsträngiger Form vor, werden sie durch Erhitzen auf Temperaturen über +50°C, bevorzugt zwischen +90°C und +100°C denaturiert (vgl. Anspruch 3). Durch schnelles Abkühlen auf eine Temperatur unter +45°C, bevorzugt unter +10°C, kann die Renaturierungszeit stark verlängert werden, so daß die Proben auch eine gewisse Zeit in einzelsträngiger Form aufbewahrt werden können, bis sie der Hybridisierungsmixtur zugesetzt werden. Die optimale Denaturierungstemperatur für doppelsträngige Proben hängt u.a. von der Art des Mediums ab, in dem sich die Proben befinden. In physiologischer Kochsalzlösung beispielsweise beträgt das Optimum zwischen +94°C und +98°C.

In bestimmten Fällen, beispielsweise bei der Hybridisierung von sogenannten singlecopy-Sequenzen ist es vorteilhaft, die gesamte Hybridisierungsmixtur inclusive Zielobjekt und Proben auf Temperaturen zwischen 50°C und 100°C, bevorzugt zwischen +70°C und +98°C, insbesondere zwischen +80°C und +98°C zu erhitzen (vgl. Anspruch 3). Die eigentliche ISH findet dann während der Abkühlung auf Temperaturen zwischen +2°C und +45°C, bevorzugt zwischen +2°C und +37°C, insbesondere zwischen +18°C und +28°C statt. Bei Untersuchung von repetitiven Sequenzen (z.B. Satellit II/III), was in der Praxis meistens der Fall ist, beträgt die Dauer der Hybridisierungsphase bevorzugt zwischen 15 s und 5 min, insbesondere zwischen 20 s und 1 min (Vgl. Anspruch 4). Werden sogenannte Single-Copy-Sequenzen untersucht, kann die Dauer der Hybridisierungsphase allerdings bis zu drei Tagen betragen, um aussagekräftige Markierungen zu bekommen.

Von besonderer Bedeutung ist für die vorliegende Erfindung, daß die Lebensfähigkeit von Zielobjektzellen nicht entscheidend durch das bei Niedertemperatur durchgeführte Verfahren beeinträchtigt wird. Zur besseren Bindung an doppelsträngige native spezifische Sequenzabschnitte der Zielobjekt-DNS können auch Proben mit geeignet modifiziertem oder ersetztem Zncker-Phosphat-Rückgrat verwendet werden. Sequenz bedeutet hier ein Molekül mit einer bestimmten Reihenfolge der Basen, sei sie nativ oder synthetisch, mit normalem, modifiziertem oder ersetztem Zucker-Phosphat-Rückgrat. Das natürliche Zucker-Phosphat-Rückgrat von Nukleinsäuren kann beispielsweise durch eine Polyamidkette ersetzt werden. Die dadurch erhältlichen Polyamidnukleinsäuren (PNS) hybridisieren aufgrund ihrer speziellen physikalischen Eigenschaften besser mit Zielobjekten als herkömmliche Nukleinsäuren.

Unabhängig von der Natur der verwendeten Probe ist es notwendig, daß sie in irgendeiner Art markiert ist, um sie nach erfolgter Hybridisierung detektieren bzw. lokalisieren zu können. Hierzu wird bevorzugt ein Fluoreszenzmarker, wie z.B. FITC, verwendet, es können aber auch alle anderen Markierungsverfahren ingesetzt werden, wie z.B. Biotinmarkierung, radioaktive Markierung oder dergleichen (vgl. Anspruch 9). Es kann auch sinnvoll sein, für eine ISH verschiedene Proben mit unterschiedlichen Markierungen zu verwenden.

Das wässrige Medium, in dem die ISH durchgeführt wird, ist bevorzugt eine physiologische Kochsalzlösung (vgl. Anspruch 6). Diese kann zusätzlich Puffersubstanzen, wie beispielsweise Tris-HCl, HEPES oder Kaliumhydrogenphosphat enthalten mit einem pH-Wert zwischen 5,0 und 8,7.

Vorteilhaft ist auch, daß das Medium zwischen 1 und 50mmol/l, insbesondere 3 mmol/l MgCl₂ enthält (vgl. Anspruch 7) sowie gegebenenfalls Gelatine oder andere Substanzen zur Erhöhung der Viskosität des Mediums, da dies die Amplifikation der Proben erleichtert. In einer weiteren Ausführungsform des Mediums für die ISH sind zusätzlich Detergenzien enthalten (vgL Anspruch 8).

Die Amplifikation der Proben, die bevorzugt mittels PCR erfolgt, kann in einem separaten Arbeitsgang erfolgen oder auch in einem wässrigem Medium, in dem auch die ISH durchgeführt wird (vgl. Anspruch 11). Während der Amplifikation der Probe kann gleichzeitig deren Markierung erfolgen.

Eine wesentliche Erweiterung und Vereinfachung der Möglichkeiten der Chromosomen-Diagnostik in sich teilenden Zellen (=Mitose), aber auch in sich nicht teilenden Zellen (=Interphase/Zellkern) ist mit dem erfindungsgemäßen Verfahren möglich. Das neue Verfahren erlaubt die vereinfachte selektive Markierung von ganzen Chromosomen, Chromosomenabschnitten und Genen bis hinunter zu wenigen hundert Basenpaaren Länge.

Eine besonders wichtige Anwendung stellt die Verwendung von ISH-Verfahren in der klinischen Tumordiagnostik inklusive Therapieplanung, Prognostik, Therapiekontrolle dar. Es wird allgemein erwartet, daß die ISH-Methoden eine wesentlich weitere Verbreitung finden und sich zu Standardmethoden der klinischen Laboratoriumsdiagnostik entwickeln werden.

Vorteilhaft ist, daß als Proben im Organismus natürlicherweise vorgefundene Sequenzen in Verbindung mit physiologischen Bedingungen des Mediums benutzt werden. Die schonenden Bedingungen des neuartigen ISH-Verfahrens erlauben auch dessen Durchführung direkt im lebenden Organismus. Dies verspricht insbesondere in der Tumortherapie große Bedeutung zu erlangen, beispielsweise zur gezielten Blockierung von Genaktivitäten.

Die Verfahren zur ISH dienen auch zum Nachweis von extrachromosomalen Strukturen, wie beispielsweise in Mitochondrien, Plastiden, Viren oder sogenannten double-minutes. Letztere besitzen eine besondere klinische Relevanz.

Die wesentlichen Vorteile des neuartigen ISH-Verfahrens ohne Zusatz von denaturierenden Agenzien zur Hybridisierungsmixtur werden im folgenden kurz zusammengefasst:
- Vereinfachung des gesamten ISH-Verfahrens durch Wegfall von bisher notwendigen Verfahrensschritten, wie z.B. Waschschritten.
- Verkürzung des gesamten ISH-Verfahrens, wodurch zuverlässige Resultate bereits nach wenigen Minuten erhalten werden.
- Schonendere Behandlung des Untersuchungsmaterials, dessen morphologische Intaktheit dadurch wesentlich besser erhalten bleibt.
- Möglichkeit der schonenden Durchführung von ISH-Verfahren, wodurch prinzipiell auch die Durchführung dieser Verfahren im lebenden Organismus möglich wird.
- Möglichkeit der gleichzeitigen Durchführung von ISH-Verfahren und anderen Testverfahren, beispielsweise mit immunologischen Techniken, in ein und dem selben Medium.

Das nachstehend beschriebene Verfahren dient zur Durchführung von ISH von spezifischen Proben, insbesondere Nukleinsäuren oder PNS-Proben oder anderen für Hybridisierungen geeigneten Proben, die eine spezifische Sequenz von Nukleotiden aufweisen, wobei die Proben mit modifizierten Nukleotiden markiert und durch ISH mit den spezifischen Stellen des Zielobjekts hybridisiert werden. Ganz allgemein beschrieben werden bei dem erfindungsgemäßen Verfahren in einem geeigneten wässrigen Medium, beispielsweise einer Pufferflüssigkeit mit einem pH-Wert zwischen 5,0 und 8,7, oder auch einer physiologischen Kochsalzlösung, im folgenden einfach als Medium bezeichnet, bei Temperaturen zwischen +2°C und +45°C (Niedertemperaturhybridisierung), gegebenenfalls nach vorheriger Erhitzung der Hybridisierungsmixtur auf Temperaturen zwischen +50° und +100°C (Hochtemperaturhybridisierung) eine oder mehrere markierte Proben, auch mit mehreren unterschiedlichen Markierungen, mit dem zu untersuchenden Zielobjekt, beispielsweise Chromosomen, ganzen Zellen oder Geweben zusammengebracht. Es erfolgt gegebenenfalls eine spezifische Hybridisierung der Proben mit komplementären Sequenzen des Zielobjekts. Nicht hybridisierte Proben werden anschließend durch Waschen entfernt. Danach wird durch ein geeignetes Verfahren, beispielsweise mittels Fluoreszenzmikroskopie und/oder Flußcytometrie die gegebenenfalls durch die Probe ans Zielobjekt hybridisierte Markierung detektiert und/oder lokalisiert. Bei einer ISH zur Blockierung von Genaktivitäten entfällt der letztgenannte Schritt. Erfindungswesentlich ist, daß während des gesamten Verfahrens denaturierende Agenzien nicht oder nur in Konzentrationen unter 10% in der Hybridisierungsmixtur anwesend sind.

Im folgenden wird das neuartige ISH-Verfahren unter Verwendung von repetitiven DNS Proben (Satellit II/III) anhand von Ausführungsbeispielen sowohl zur Hochtemperatur-, als auch zur Niedertemperatur-ISH näher erläutert.

### Hochtemperatur-ISH:

### Beispiel I:

### Verwendete Chemikalien:

- Proben DNS markiert (immunofluoresenzmarkiert) 1 - 70 ng
- Hybridisierungspuffer 10x : 3 µl
   (Tris-HCl :1-200 mmol/l; MgCl₂: 1-50 mmol/l;
   KCl : 1-600 mmol/l; Gelatine: 1-10 mg/l)
- Natriumcitrat, gesättigt, 20x : 3 µl
- H₂O zum Erreichen des Endvolumens von 30 µl

Endvolumina (30µl) werden auf Objektträger mit fixierten Metaphasechromosomen (Zielobjekt-DNS) aufgetragen und luftdicht verschlossen. Inkubation der Proben-DNS und der Zielobjekt-DNS bei 95°C für 5 min. Danach Inkubation bei 40°C für 30 min. Danach Objektträger 1x waschen in physiologischer Kochsalzlösung, Tween 20 (0.2 vol%) für 5 min bei Raumtemperatur. Danach lufttrocknen und Antikörperkonjugat gelöst in physiologischer Kochsalzlösung, Tween 20 (0.2 vol%) auftragen, luftdicht verschließen und im Dunkeln bei Raumtemperatur inkubieren.
Bei "direkter Fluoreszenz" entfällt die Antikörperanbindung nach erfolgter Waschung in physiologischer Kochsalzlösung, Tween 20 (0.2 vol%).
Die Objektträger können jetzt einer Analyse unterzogen werden.

### Beispiel II:

### Verwendete Chemikalien:

- Proben DNS markiert (immunofluoreszenzmarkiert): 1 - 70 ng
- Physiologische Kochsalzlösung zum Erreichen des Endvolumens von 30 µl.
Die Durchführung des Verfahrens erfolgt wie unter Beispiel 1 beschrieben.

### Niedertemperatur-ISH:

### Beispiel III:

### Verwendete Chemikalien:

- Proben DNS markiert (immunofluoreszenzmarkiert): 1 - 70 ng
- Physiologische Kochsalzlösung zum Erreichen des Endvolumens von 30 µl.
Vor dem Auftragen auf die Objektträger mit den fixierten Chromosomen (Zielobjekt-DNS) werden die Proben in physiologischer Kochsalzlösung einer Temperaturbehandlung von 5min Dauer bei 95°C unterzogen und dann sofort auf -20°C abgekühlt. Bei Zimmertemperatur (+20°C) werden die Proben dann auf die Objektträger mit den fixierten Chromosomen (Zielobjekt DNS) aufgetragen, luftdicht verschlossen und für 10 Stunden bei 37°C inkubiert. Weitere Schritte für Immunofluoreszenz bzw. direkte Fluoreszenz siehe Beispiel I und II.

Auch für alphoide Proben DNS (D15Z1) wurden diese Protokolle erfolgreich eingesetzt.

In der Praxis können zur Durchführung des neuartigen ISH-Verfahrens entsprechende Testkits angeboten werden. Bevorzugt besteht ein solches Testkit aus mindestens einem wässrigen Medium und aus Proben, insbesondere aus amplifizierten, und/oder markierten oder anderen künstlich hergestellten Proben. Es kann für das Testkit eine Waschlösung vorgesehen sein, insbesondere eine Waschlösung, die keine denaturierenden Agenzien enthält. Die Proben sind markiert, insbesondere durch Einbau modifizierter Nukleotide für direkte oder indirekte Immunofluoreszenz oder radioaktive Gruppen, durch Kopplung an geeignete Fluorochrome oder durch andere, in der Biochemie gängige Methoden. Entscheidend für diese Markierung ist, daß nach erfolgter ISH die spezifisch gebundenen Proben detektiert und lokalisiert werden können. In einer bevorzugten Ausführungsform enthält der Testkit auch ein oder mehrere Reaktionsgefäße.

Das erfindungsgemäße ISH-Verfahren ist, wie beschrieben, geeignet zur Kombination mit immunologischen Färbeverfahren, wobei beide Verfahren simultan und in ein und demselben Medium durchgeführt werden können. Ein entsprechendes Testkit enthält neben den für das ISH-Verfahren notwendigen Bestandteilen wie Proben und gegebenenfalls Puffer, Waschlösung und Reaktionsgefäßen mindestens einen Antikörper, der gegen spezifische zelluläre Antigene gerichtet und gegebenenfalls markiert ist. Weiterhin kann ein solches Testkit auch einen sekundären Antikörper enthalten, der gegen den primären Antikörper gerichtet und markiert ist. Hierzu ist es vorteilhaft, daß das die einzelnen Bestandteile enthaltende wässrige Medium eine physiologische Kochsalzlösung ist oder ein Puffer, wie er auch für die Polymerase Kettenreaktion (PCR) verwendet werden kann, wobei zusätzlich gesättigtes Natriumcitrat zugesetzt werden kann (vgl Anspruch 8).

Werden die Proben für die ISH in einzelsträngiger Form angeboten, können sie zusammen mit den primären Antikörpern für den immunologischen Test aufbewahrt werden. In diesem Fall könnte das gesamte Testkit aus einem einzigen Gefäß bestehen, enthaltend:
- Wässriges Medium, z.B. physiologische Kochsalzlösung
- Proben
- Antikörper

Ein Beispiel für ein Testkit für die neuartige ISH ist nachfolgend beschrieben. Es besteht aus vier Gefäßen. Die Gefäße 2 und 3 enthalten Puffer, das Gefäß 1 die Proben-DNS und das Gefäß 4 die physiologische Kochsalzlösung.

Gefäß Nr. 1 enthält eine beliebige Menge schon markierter (z.B. direkt fluoreszenzmarkierter) Proben in H₂O.

Gefäß Nr. 2 enthält Hybridisierungspuffer:

| | |
|---|---|
| Tris-HCl | 1-200 mmol/l |
| MgCl₂ | 1-50 mmol/l |
| KCl | 1-600 mmol/l |
| Gelatine | 1-10 mg/l |

Anstatt KCl und Gelatine ist im Gefäß Nr. 3: Polyoxyethylene Sorbitan Monooleate (Tween 20) 0,1-50 mg/l und Octylphenol Ethylene Oxyde Kondensate 0,1-50mg/l enthalten.

Anstatt des Hybridisierungspuffers gemäß dem Inhalt im Gefäß Nr. 2 oder 3 kann auch eine physiologische Kochsalzlösung enthalten sein.

Vorteilhaft ist es, wenn im Puffer (Gefäß 2, 3) gesättigtes Natriumcitrat enthalten ist.

Gefäß 4 enthält einen für Immunfluoreszenz markierten Antikörper in physiologischer Kochsalzlösung/Tween 20.

Das Kit besteht aus den vier Gefäßen Nr. 1 bis 4. Für den Waschschritt nach der Hybridisierung muß zusätzlich noch physiologische Kochsalzlösung (0,9 vol% NaCl) selbst hergestellt werden. Diese Waschlösung kann zusätzlich 0,2 vol% Polyoxyethylene Sorbitan Monooleate enthalten.

Auch die Antikörperanbindung bei indirekter Immunofluoreszenz wird in physiologischer Kochsalzlösung vorgenommen, und zwar mit oder ohne Polyoxyethylene Sorbitan Monooleate (Tween 20). Der/die Waschschritt/e nach Antikörperanbindung erfolgt/en in physiologischer Kochsalzlösung.

## Patentansprüche

1. Verfahren zur In-situ-Hybridisierung von spezifischen Proben, insbesondere Nukleinsäuren oder Polyamidnukleinsäuren oder anderen Proben, die eine spezifische Sequenz von Nukleotiden aufweisen und mit modifizierten Nukleotiden markiert sind, an Nukleinsäuren enthaltende Zielobjekte, nämlich eukaryontische chromosomale und extrachromosomale Strukturen, in einem wässrigen Medium bzw. Puffer **dadurch gekennzeichnet,**
- **daß** die Zielobjekte ohne denaturierende Vorbehandlung eingesetzt werden,
- **daß** die Proben als Einzel- und/oder Doppelstränge eingesetzt werden, wobei die Einzelstränge originär erzeugt oder sekundär durch Hitzedenaturierung aus ursprünglichen Doppelsträngen hergestellt sind,
- **daß** das wässrige Medium bzw. der Puffer frei von denaturierenden die Denaturierung fördernden oder erhaltenden chemischen Agenzien und Enzymen in Mengen, die für eine Behandlung zur Erzeugung von einzelsträngigen DNS-Abschnitten geeignet sind, ist,
- **daß** Zielobjekte, Proben und wässriges Medium zur Hybridisierungsmixtur miteinander vermischt werden, und
- **daß** die Hybridisierungsphase bei einer Temperatur zwischen +2°C und +100°C durchgeführt wird, wobei die Hybridisierungsmixtur frei ist von denaturierenden die Denaturierung fördernden oder erhaltenden chemischen Agenzien und Enzymen in Mengen, die für eine Behandlung zur Erzeugung von einzelsträngigen DNS-Abschnitten geeignet sind

2. Verfahren nach Anspruch 1, **dadurch gekennzeichnet, daß** die in situ Hybridisierung von Proben an Zielobjekte bei Temperaturen zwischen +2°C und +45°C, insbesondere zwischen +2°C und +37°C, ganz bevorzugt zwischen +18°C und +28°C erfolgt.

3. Verfahren nach Anspruch 1, **dadurch gekennzeichnet, daß** die Proben vor der eigentlichen in situ Hybridisierung erhitzt werden auf Temperaturen zwischen +50°C und +100°C, insbesondere zwischen +70°C und +98°C, ganz bevorzugt zwischen +80 und +98°C und anschließend abgekühlt werden auf Temperaturen zwischen +2°C und +45°C, insbesondere zwischen +2°C und +37°C, ganz bevorzugt zwischen +18°C und +28°C.

4. Verfahren nach Anspruch 3, **dadurch gekennzeichnet, daß** der Abkühlvorgang zwischen 15 s und 5 min, insbesondere zwischen 20 s und 1 min dauert.

5. Verfahren nach Anspruch 1 bis 4, **dadurch gekennzeichnet, daß** die Proben einzelsträngig sind.

6. Verfahren nach Anspruch 1 bis 5, **dadurch gekennzeichnet, daß** das wässrige Medium eine physiologische Kochsalzlösung ist.

7. Verfahren nach Anspruch 1 bis 6, **dadurch gekennzeichnet, daß** das wässrige Medium zwischen 1 und 50 mmol/l, insbesondere 3 mmol/l MgCl₂ und/oder eine Puffersubstanz und gegebenenfalls eine Substanz zur Erhöhung der Viskosität, wie beispielsweise Gelatine, Polyoxyethylen Sorbitan Monooleat oder Octylphenol Ethylenoxid Kondensat, enthält.

8. Verfahren nach Anspruch 1 bis 7, **dadurch gekennzeichnet, daß** das wässrige Medium Natriumcitrat und/oder Detergentien enthält.

9. Verfahren nach Anspruch 1 bis 8, **dadurch gekennzeichnet, daß** die Proben eine geeignete Markierung, beispielsweise Fluorochrome, Biotin, oder dergleichen, tragen.

10. Verfahren nach Anspruch 1 bis 9, **dadurch gekennzeichnet, daß** als Probe ein oder mehrere Antikörper verwendet werden, die spezifische Nukleinsäuresequenzen erkennen.

11. Verfahren nach einem oder mehreren der vorangegangenen Ansprüche, **dadurch gekennzeichnet, daß** die Amplifikation der Proben und die eigentliche in situ Hybridisierung in ein und demselben wässrigen Medium stattfindet.

12. Verfahren nach einem oder mehreren der vorangegangenen Ansprüche, **dadurch gekennzeichnet, daß** zur sequenzspezifischen Anlagerung von Proben an zelluläre Zielobjekte als Proben im Organismus natürlicherweise vorgefundene Nukleinsäuresequenzen in Verbindung mit physiologischen Bedingungen des Mediums benutzt werden.

13. Verfahren nach einem oder mehreren der vorangegangenen Ansprüche, **dadurch gekennzeichnet, daß** zelluläre Untersuchungsobjekte gleichzeitig und in ein und demselben wässrigen Medium durch in situ Hybridisierung und immunologische Techniken auf spezifische Oberflächenantigene und numerische bzw. strukturelle Chromosomenveränderungen untersucht werden.

14. Verfahren nach einem oder mehreren der vorangegangenen Ansprüche, **dadurch gekennzeichnet, daß** die Auswertung der eingeführten Markierungen durch mikroskopische Beobachtung und/oder digitale Mikroskopie und/oder Durchflußcytometrie oder dergleichen erfolgt.

15. Verfahren nach einem oder mehreren der vorhergehenden Ansprüche, **dadurch gekennzeichnet, daß** das Verfahren direkt in Kulturen lebender Zellen durchgeführt wird.

16. Verwendung eines Kits bestehend aus
- mindestens einem Gefäß, das spezifische, markierte Proben, bevorzugt bereits amplifizierte Proben, enthält und/oder
- mindestens einer speziellen Pufferlösung und/oder
- mindestens einer speziellen Waschlösung und/oder
- einem Reaktionsgefäß und/oder
- Antikörpern zur Durchführung eines Verfahrens nach Anspruch 1 bis 15.

17. Verwendung nach Anspruch 16 mit Antikörpern, **dadurch gekennzeichnet daß** die Antikörper markiert sind, beispielsweise durch Fluorochrome, Biotin, oder dergleichen.

18. Verwendung nach Anspruch 16 oder 17 mit Antikörpern, **dadurch gekennzeichnet daß** das Testkit grimäre und sekundäre Antikörper enthält, wobei der sekundäre Antikörper gegen den primären Antikörper gerichtet und markiert ist.

## Claims

1. Process for in-situ hybridisation of specific probes, in particular nucleic acids or polyamide nucleic acids or other probes which have a specific sequence of nucleotides and are marked with modified nucleotides, to target objects containing nucleic acids, namely eukaryotic chromosomal and extra-chromosomal structures, in an aqueous medium or buffer, **characterised in that**
- the target objects are used without denaturing pretreatment,
- the probes are used as single strands and/or double strands, wherein the single strands are produced originally or are made secondarily by thermal denaturing from original double strands,
- the aqueous medium or the buffer is free of denaturing chemical agents which promote or preserve denaturing and enzymes in quantities which are suitable for a treatment for producing single-stranded DNA sections,
- target objects, probes and aqueous medium are mixed with one another to form the hybridising mixture, and
- the hybridising phase is carried out at a temperature between +2°C and +100°C, wherein the hybridising mixture is free of denaturing chemical agents which promote or preserve denaturing and enzymes in quantities which are suitable for a treatment for producing single-stranded DNA sections.

2. Process according to claim 1, **characterised in that** the in-situ hybridisationof probes to target objects takes place at temperatures between +2°C and +45°C, in particular between +2°C and +37°C, most preferably between +18°C and +28°C.

3. Process according to claim 1, **characterised in that** the probes are heated at temperatures between +50°C and +100°C, in particular between +70°C and +98°C, most preferably between +80 and +98°C before the actual in-situ hybridisation and are then cooled to temperatures between +2°C and +45°C, in particular between +2°C and +37°C, most preferably between +18°C and +28°C.

4. Process according to claim 3, **characterised in that** the cooling process lasts between 15 seconds and 5 minutes, in particular between 20 seconds and 1 minute.

5. Process according to claim 1 to 4, **characterised in that** the probes are single-stranded.

6. Process according to claim 1 to 5, **characterised in that** the aqueous medium is a physiological saline solution.

7. Process according to claim 1 to 6, **characterised in that** the aqueous medium contains between 1 and 50 mmoles/litre, in particular 3 mmoles/litre, of MgCl₂ and/or a buffer substance and optionally a substance for increasing the viscosity, such as for example gelatine, polyoxyethylene sorbitan monooleate or octylphenol ethylene oxide condensate.

8. Process according to claim 1 to 7, **characterised in that** the aqueous medium contains sodium citrate and/or detergents.

9. Process according to claim 1 to 8, **characterised in that** the probes carry a suitable marking, for example fluorochromes, biotin or the like.

10. Process according to claim 1 to 9, **characterised in that** one or more antibodies, which recognise specific nucleic acid sequences, are used as the probe.

11. Process according to one or more of the preceding claims, **characterised in that** amplification of the probes and the actual in-situ hybridisation takes place in one and the same aqueous medium

12. Process according to one or more of the preceding claims, **characterised in that** for sequence-specific attachment of probes to cellular target objects, naturally previously found nucleic acid sequences are used as probes in the organism in conjunction with physiological conditions of the medium.

13. Process according to one or more of the preceding claims, **characterised in that** cellular investigation objects are investigated at the same time and in one and the same aqueous medium by in-situ hybridisation and immunological techniques on specific surface antigens and numerical or structural chromosome changes.

14. Process according to one or more of the preceding claims, **characterised in that** evaluation of the markings introduced takes place by microscopic observation and/or digital microscopy and/or flow cytometry or the like.

15. Process according to one or more of the preceding claims, **characterised in that** the process is carried out directly in cultures of living cells.

16. Use of a kit consisting of
- at least one vessel, which contains specific, labelled probes, preferably already amplified probes, and/or
- at least one special buffer solution and/or
- at least one special washing solution and/or
- a reaction vessel and/or
- antibodies
for carrying out the process according to claim 1 to 15.

17. Use according to claim 16 with antibodies, **characterised in that** the antibodies are labelled, for example by fluorochromes, biotin or the like.

18. Use according to claim 16 or 17 with antibodies, **characterised in that** the test kit contains primary and secondary antibodies, wherein the secondary antibody is directed and labelled against the primary antibody.

## Revendications

1. Procédé d'hybridation in situ de sondes spécifiques, en particulier d'acides nucléiques ou d'acides polyamide-nucléiques ou d'autres sondes qui présentent une séquence spécifique de nucléotides et qui sont marquées par des nucléotides modifiés, avec des objets cibles contenant des acides nucléiques, à savoir des structures chromosomiques et extrachromosomiques eucaryotes, dans un milieu aqueux ou un tampon, **caractérisé**
- **en ce que** les objets cibles sont utilisés sans traitement préalable de dénaturation,
- **en ce que** les sondes sont utilisées en tant que sondes simple brin et/ou double brin, les sondes simple brin étant générées à l'origine ou fabriquées de façon secondaire par dénaturation à la chaleur à partir de doubles brins originaux,
- **en ce que** le milieu aqueux ou le tampon est exempt d'agents chimiques dénaturants, favorisant ou soutenant la dénaturation, et d'enzymes dans des quantités qui seraient appropriées pour un traitement permettant la génération de segments d'ADN simple brin,
- **en ce que** des objets cibles, des sondes et du milieu aqueux sont mélangés ensemble pour former un mélange d'hybridation, et
- **en ce que** la phase d'hybridation est conduite à une température comprise entre +2 °C et + 100 °C, le mélange d'hybridation étant exempt d'agents chimiques dénaturants, favorisant ou soutenant la dénaturation, et d'enzymes dans des quantités qui seraient appropriées pour un traitement permettant la génération de segments d'ADN simple brin.

2. Procédé selon la revendication 1, **caractérisé en ce que** l'hybridation in situ de sondes avec des objets cibles est réalisée à des températures comprises entre + 2 °C et + 45 °C, en particulier entre + 2 °C et + 37 °C, de façon tout à fait préférée entre + 18 °C et + 28 °C.

3. Procédé selon la revendication 1, **caractérisé en ce que** les sondes sont chauffées avant l'hybridation in situ à proprement parler à des températures comprises entre + 50 °C et + 100 °C, en particulier entre + 70 °C et + 98 °C, de façon tout à fait préférée entre + 80 °C et + 98 °C, puis sont refroidies à des températures comprises entre + 2 °C et + 45 °C, en particulier entre + 2 °C et + 37 °C, de façon tout à fait préférée entre + 18 °C et + 28 °C.

4. Procédé selon la revendication 3, **caractérisé en ce que** le processus de refroidissement dure entre 15 s et 5 minutes, en particulier entre 20 s et 1 minute.

5. Procédé selon la revendication 1 à 4, **caractérisé en ce que** les sondes sont des sondes simple brin.

6. Procédé selon la revendication 1 à 5, **caractérisé en ce que** le milieu aqueux est une solution physiologique de chlorure de sodium.

7. Procédé selon la revendication 1 à 6, **caractérisé en ce que** le milieu aqueux contient 1 à 50 mmoles/l, en particulier 3 mmoles/l de MgCl₂ et/ou une substance tampon et éventuellement une substance permettant d'accroître la viscosité, comme par exemple de la gélatine, du monooléate polyoxyéthylénique de sorbitane ou un condensat d'octylphénol-oxyde d'éthylène.

8. Procédé selon la revendication 1 à 7, **caractérisé en ce que** le milieu aqueux contient du citrate de sodium et/ou des détersifs.

9. Procédé selon la revendication 1 à 8, **caractérisé en ce que** les sondes portent un marquage approprié, par exemple des fluorochromes, de la biotine ou équivalent.

10. Procédé selon la revendication 1 à 9, **caractérisé en ce que** l'on utilise comme sonde un ou plusieurs anticorps reconnaissant les séquences d'acides nucléiques spécifiques.

11. Procédé selon une ou plusieurs des revendications précédentes, **caractérisé en ce que** l'amplification des sondes et l'hybridation in situ à proprement parler se fait dans un seul et même milieu aqueux.

12. Procédé selon une ou plusieurs des revendications précédentes, **caractérisé en ce que**, pour la fixation spécifique à la séquence de sondes sur des objets cibles cellulaires, on utilise comme sondes des séquences d'acides nucléiques qui se trouvent naturellement dans l'organisme en liaison avec des conditions physiologiques du milieu.

13. Procédé selon une ou plusieurs des revendications précédentes, **caractérisé en ce que** l'on analyse, des objets d'analyse cellulaires, simultanément et dans un seul et même milieu aqueux par hybridation in situ et par des techniques immunologiques, des antigènes de surface spécifiques et des modifications chromosomiques numériques ou structurelles.

14. Procédé selon une ou plusieurs des revendications précédentes, **caractérisé en ce que** l'évaluation des marquages introduits se fait par examen au microscope et/ou par microscopie numérique et/ ou par cytométrie en flux, ou analogues.

15. Procédé selon une ou plusieurs des revendications précédentes, **caractérisé en ce que** le procédé est directement réalisé dans des cultures de cellules vivantes.

16. Utilisation d'un kit composé de
- au moins un réceptacle, qui contient des sondes spécifiques marquées, de préférence des sondes déjà amplifiées, et/ou
- au moins une solution tampon spéciale et/ou
- au moins une solution de lavage spéciale et/ou
- un réceptacle de réaction et/ou
- des anticorps,
pour réaliser un procédé selon la revendication 1 à 15.

17. Utilisation selon la revendication 16 avec des anticorps, **caractérisé en ce que** les anticorps sont marqués, par exemple par des fluorochromes, de la biotine ou analogues.

18. Utilisation selon la revendication 16 ou 17 avec des anticorps, **caractérisé en ce que** le kit de test contient des anticorps primaires et secondaires, l'anticorps secondaire étant dirigé contre l'anticorps primaire et marqué.
